# FASCICULE DE BREVET EUROPEEN

(11) **EP 1 060 157 B1**
(45) Date de publication et mention de la délivrance du brevet: **25.06.2003**
(21) Numéro de dépôt: 99937924.1
(22) Date de dépôt: 24.02.1999
(51) Int. Cl.: C07C 51/44, C07C 51/487, C07C 51/43, C07C 51/42, C07C 55/14, C07C 51/31

(54) **PROCEDE DE SEPARATION ET DE PURIFICATION DE L'ACIDE ADIPIQUE**
VERFAHREN ZUR TRENNUNG UND REINIGUNG VON ADIPINSÄURE
METHOD FOR SEPARATING AND PURIFYING ADIPIC ACID

(30) Priorité: 05.03.1998 FR 9802928
(43) Date de publication de la demande: 20.12.2000
(73) Titulaire: Rhodia Polyamide Intermediates, 69192 Saint-Fons (FR)
(72) Inventeur: COSTANTINI, Michel, F-69003 Lyon (FR); FACHE, Eric, F-69300 Caluire et Cuire (FR); LECONTE, Philippe, F-69330 Meyzieu (FR)
(74) Mandataire: Esson, Jean-Pierre
(86) Numéro de dépôt international: FR9900420
(87) Numéro de publication internationale: WO99044980

(56) Documents cités:
- WO-A-96/03365
- WO-A-97/36673
- DE-A- 4 428 977
- DE-C- 764 488
- FR-A- 1 266 886
- FR-A- 2 092 524
- FR-A- 2 353 513
- FR-A- 2 390 415
- FR-A- 2 757 155
- US-A- 3 933 930

## Description

La présente invention concerne le traitement des mélanges réactionnels issus d'une réaction d'oxydation du cyclohexane en acide adipique et plus particulièrement la séparation des différents constituants desdits mélanges et la purification de l'acide adipique.

L'oxydation directe du cyclohexane en acide adipique est un procédé qui a été travaillé depuis longtemps, notamment en raison des avantages évidents qu'il y aurait à convertir le cyclohexane en acide adipique, en une seule étape et sans mettre en oeuvre un oxydant tel que l'acide nitrique, ce composé générant des oxydes d'azote qu'il faut ensuite traiter pour éviter toute pollution.

Le brevet WO-A-94/07834 décrit l'oxydation d'hydrocarbures cycliques en diacides correspondants, en phase liquide comportant un solvant, à l'aide d'un gaz contenant de l'oxygène et en présence d'un catalyseur d'oxydation tel qu'un composé du cobalt, ledit solvant comprenant un acide organique n'ayant que des atomes d'hydrogène primaires ou secondaires. Ce brevet développe plus particulièrement les phases de traitement du mélange réactionnel final. Ce traitement consiste à séparer le diacide formé, en refroidissant le mélange pour provoquer la précipitation dudit diacide, à séparer par filtration le diacide de deux phases liquides, une non-polaire qui est recyclée, une polaire qui est également recyclée après une éventuelle hydrolyse et une séparation d'une quantité supplémentaire de diacide.

Ce brevet propose une solution pour oxyder en une étape le cyclohexane en acide adipique avec une sélectivité industriellement acceptable, mais il ne fournit pas de solution applicable industriellement au traitement du mélange réactionnel issu de l'oxydation, prenant en compte la séparation des différents produits et sous-produits de la réaction, des produits non transformés et du catalyseur.

En outre, il s'avère en pratique qu'un procédé de traitement aussi sommaire ne conduit pas à un acide adipique présentant la pureté requise dans de très nombreuses applications de cette matière première très importante.

En effet, que ce soit pour la production de polyamide 6,6 ou pour d'autres applications telles que la production de certains polyuréthannes, la pureté de l'acide adipique mis en oeuvre doit être extrêmement élevée, tant pour les teneurs en sous-produits organiques pouvant amener des colorations indésirables que pour les teneurs en résidus métalliques, notamment les traces du catalyseur utilisé.

Le brevet français n° 2757 155 décrit un procédé de purification d'acide adipique obtenu par oxydation du cyclohexane. Ce procédé consiste à purifier l'acide adipique par un traitement à l'acide nitrique puis à cristalliser l'acide traité dans l'eau. Ce document précise que l'acide adipique ne doit pas être chaufé à une température supérieure à 75°C avant le traitement à l'acide nitrique. Dans le cas contraire, il est impossible d'obtenir un acide présentant les critères de puretés convenables pour une utilisation en polymérisation.

La présente invention concerne donc un procédé perfectionné de traitement du mélange réactionnel issu de l'oxydation directe du cyclohexane en acide adipique par
- une décantation en deux phases liquides : une phase supérieure essentiellement cyclohexanique et une phase inférieure comprenant essentiellement le solvant, les diacides formés, le catalyseur et une partie des autres produits de la réaction et du cyclohexane non transformé ;
- une distillation de ladite phase inférieure, permettant de séparer d'une part un distillat comprenant au moins une partie des composés les plus volatils tels que le solvant organique, l'eau ainsi que le cyclohexane non transformé, la cyclohexanone, le cyclohexanol, les esters de cyclohexyle et les lactones éventuellement présents, et d'autre part le pied de distillation comprenant les diacides formés, le catalyseur ;
- une séparation du catalyseur du pied de distillation obtenu précédemment, soit par cristallisation dans l'eau, par électrodialyse ou par passage sur résine échangeuse d'ions, après dissolution dudit pied de distillation dans l'eau, soit par lavage à l'eau ou par extraction liquide-liquide ;
- un traitement de purification réducteur et/ou oxydant de l'acide adipique en solution aqueuse ;
- une cristallisation précédant ou suivant le traitement de purification lorsqu'elle n'a pas été réalisée pour séparer le catalyseur ;
- une recristallisation dans l'eau de l'acide adipique.

La phase cyclohexanique obtenue dans l'étape de décantation est le plus souvent réintroduite dans une opération d'oxydation du cyclohexane.

Le solvant organique mis en oeuvre dans l'oxydation du cyclohexane est plus particulièrement choisi parmi les acides carboxyliques aliphatiques. C'est le plus souvent l'acide acétique.

Le catalyseur contient de préférence du cobalt, du manganèse, un mélange de cobalt avec un ou plusieurs autres métaux comme le manganèse, le chrome, le fer, le zirconium, l'hafnium, le cuivre. Parmi les mélanges à base de cobalt, les catalyseurs comprenant soit du cobalt et du chrome, soit du cobalt, du chrome et du zirconium, soit du cobalt et du fer, soit du cobalt et du manganèse, soit du cobalt et du zirconium et/ou de l'hafnium conviennent plus particulièrement bien. Ce catalyseur est engagé pour l'oxydation du cyclohexane, sous la forme de composés de ces métaux solubles dans le milieu réactionnel.

L'étape de distillation de la phase inférieure est conduite de telle façon que la majeure partie et, dans la mesure du possible, la quasi-totalité du cyclohexane non transformé, pouvant être encore présent dans cette phase inférieure, et du solvant, notamment de l'acide carboxylique utilisé de préférence, soit séparée de l'acide adipique. Cette étape permet de séparer les composés organiques légers (plus volatils que les diacides) qu'il est intéressant de recycler dans l'étape d'oxydation du cyclohexane, éventuellement après un traitement destiné à les déhydrater. Comme exemples de tels composés organiques légers, on peut citer les composés adipogènes (susceptibles d'être transformés en acide adipique) tels que le cyclohexanol, la cyclohexanone, l'acétate de cyclohexyle et d'autres composés comme des lactones (butyrolactone, valérolactone essentiellement).

L'étape de distillation est généralement effectuée à une température de 25°C à 250°C et sous une pression absolue comprise entre 10 Pa et la pression atmosphérique. De préférence la température du mélange pendant la distillation sera maintenue entre 70°C et 150°C.

La distillation peut, si nécessaire, être conduite en plusieurs étapes successives, en particulier dans le mode préféré où l'on souhaite éliminer la plus grosse partie, par exemple plus de 90 % et même plus de 99 % du solvant tel qu'un acide carboxylique aliphatique.

Pour parfaire la séparation des composés organiques légers mentionnés précédemment, on peut utiliser dans la distillation un entraîneur inerte, qui peut soit être de l'eau sous forme de vapeur, soit aussi être un gaz inerte tel que l'azote.

L'étape de distillation peut être éventuellement complétée par une extraction du pied de distillation à l'aide d'un solvant organique non miscible à l'eau. Cette extraction peut servir à séparer les esters, notamment de cyclohexyle, susceptibles de se trouver dans le pied de distillation. A titre d'exemples non limitatifs de tels solvants organiques, on peut utiliser des hydrocarbures aliphatiques, cycloaliphatiques ou aromatiques, des esters d'acides carboxyliques aliphatiques, cycloaliphatiques ou aromatiques, des cétones. Comme il est préférable d'éviter dans la mesure du possible l'introduction de nouveaux composés dans le procédé de l'invention, on utilisera avantageusement comme solvant d'extraction le cyclohexane. L'extrait peut être recyclé dans une nouvelle réaction d'oxydation, soit directement, soit après hydrolyse des esters.

Le distillat obtenu dans l'opération de distillation décrite précédemment comprend les différents composés volatils et de l'eau. Ces composés volatils sont valorisables et sont donc recyclés dans une nouvelle réaction d'oxydation du cyclohexane, après une élimination au moins partielle de l'eau par tout moyen connu, notamment par distillation azéotropique.

Le pied de distillation obtenu en fin de distillation, et ayant subi le cas échéant l'opération d'extraction, est traité pour séparer le catalyseur qu'il contient.

Cette séparation peut s'effectuer dans une première variante par une opération de cristallisation qui consiste essentiellement à dissoudre ledit pied de distillation dans la quantité minimale d'eau, généralement à chaud, puis à cristalliser principalement l'acide adipique. La solution aqueuse contenant le catalyseur peut être traitée par ailleurs pour isoler ledit catalyseur, pouvant être recycler dans une nouvelle opération d'oxydation.

La séparation peut aussi être réalisée par d'autres techniques connues, par exemple, après dissolution dudit pied de distillation dans de l'eau, en faisant une électrodialyse de la solution obtenue ou en passant ladite solution sur une résine échangeuse d'ions. L'électrodialyse peut être effectuée comme décrit dans la demande de brevet WO-A-97/36673. Les résines échangeuses d'ions sont des résines susceptibles de fixer les cations métalliques du catalyseur. Elles ont généralement des fonctions à caractère acide ou des fonctions à caractère complexant. Les fonctions à caractère acide sont le plus souvent des groupes acide sulfonique ou acide carboxylique. Les fonctions à caractère complexant sont le plus souvent des groupes de type imidodiacétique ou aminophosphonique.

La séparation peut aussi être effectuée dans une autre variante par un ou plusieurs lavages du pied de distillation par l'eau. Par un tel lavage, on dissout notamment le catalyseur ainsi qu'une partie des diacides, notamment l'acide glutarique et à un moindre degré l'acide succinique. Pour éviter ou limiter fortement la dissolution d'acide adipique, on met en oeuvre une quantité d'eau ou d'eau saturée en acide adipique qui représente en poids de 1 % à 100 % du poids du pied de distillation et de préférence de 10 % à 50 %.

L'étape de séparation du catalyseur est suivie par une opération de purification de l'acide adipique qui a été mis en solution aqueuse.

Cette purification peut être réalisée par hydrogénation et/ou par traitement à l'acide nitrique et/ou par oxydation par l'oxygène moléculaire ou par tout autre oxydant tel que l'ozone et les hydroperoxydes (incluant le peroxyde d'hydrogène).

L'hydrogénation est avantageusement effectuée à l'aide d'hydrogène et en présence d'un catalyseur. Comme catalyseur, on peut citer à titre d'exemples non limitatifs ceux qui contiennent au moins un métal du groupe 8 de la Classification périodique des éléments tel que le palladium, le platine, le ruthénium, l'osmium, le rhodium, l'iridium, le nickel, le cobalt. Ces métaux sont de préférence sous forme métallique et sont avantageusement déposés sur un support solide. Comme support solide, on peut utiliser non limitativement des charbons, des argiles, des zéolithes, des oxydes tels que les silices, les alumines, les silices-alumines, la magnésie. Le catalyseur peut être mis en oeuvre en lit fixe ou en lit fluidisé ou transporté. L'hydrogénation peut être réalisée en continu ou en discontinu, mais la marche continue est privilégiée dans la perspective d'une installation de type industriel.

Le traitement à l'acide nitrique peut être réalisé avec une solution aqueuse contenant généralement de 20 % à 80 % d'acide nitrique pur en poids par poids de solution. Ce traitement est généralement effectué en chauffant le mélange à une température de 25°C à 120°C et de préférence de 40°C à 100°C pendant une durée de quelques minutes à quelques heures. Le chauffage peut avantageusement être conduit par paliers successifs à une température située dans les zones de valeurs indiquées précédemment. La quantité d'acide nitrique mis en oeuvre peut varier largement. Elle doit d'une part bien évidemment être suffisante pour l'oxydation souhaitée et d'autre part ne pas être trop importante pour des raisons à la fois techniques et économiques. Généralement on mettra en oeuvre de 0,8 mole à 4 moles, et de préférence de 1 mole à 2 moles, d'acide nitrique pour 100 grammes de solution d'acide adipique à traiter. Ce traitement est généralement réalisé en l'absence de catalyseur. Il peut également être conduit en présence d'un catalyseur, comprenant un ou plusieurs composés de cobalt, de cuivre et/ou de vanadium. Le traitement pouvant générer la formation de vapeurs nitreuses, il est de préférence complété par une élimination à chaud desdites vapeurs nitreuses formées, par circulation dans le mélange liquide d'un gaz inerte, tel que l'azote.

L'oxydation par l'oxygène moléculaire est plus particulièrement réalisée avec de l'air, de l'air enrichi en oxygène ou de l'air appauvri en oxygène et en présence d'un catalyseur. Les catalyseurs décrits précédemment pour le traitement par hydrogénation conviennent pour ce traitement par l'oxygène moléculaire. De préférence on peut utiliser un métal choisi parmi le palladium, le platine, le ruthénium, l'osmium, le rhodium, l'iridium.

L'oxydation par les hydroperoxydes, réalisée de préférence avec le peroxyde d'hydrogène, peut être non catalysée ou être catalysée par les catalyseurs classiquement utilisés avec ce type d'oxydant. On préfère plus particulièrement les catalyseurs hétérogènes de type tamis moléculaire. On peut par exemple se référer aux catalyseurs décrits dans la demande de brevet WO 96/31455 ou dans le brevet FR-A-2 744 719. On peut en particulier avantageusement utiliser ceux qui contiennent du titane.

La purification par hydrogénation et/ou traitement nitrique et/ou oxydation à l'aide d'oxygène moléculaire, d'ozone ou d'hydroperoxyde peut être précédée ou suivie d'un traitement d'adsorption d'impuretés par un solide finement divisé tel que par exemple un noir de carbone ou une alumine. Ce traitement comprend schématiquement l'addition de noir de carbone ou d'alumine à la solution aqueuse chaude contenant l'acide adipique et la filtration à chaud de ladite solution pour séparer le noir de carbone et les impuretés adsorbées. Ce traitement peut également être réalisé en continu sur un lit fixe.

Le traitement par hydrogénation et/ou le traitement à l'acide nitrique et/ou le traitement par oxydation à l'aide d'oxygène moléculaire, d'ozone ou d'hydroperoxyde est généralement suivi par une opération de cristallisation de l'acide adipique dans l'eau, permettant notamment de séparer les acides glutarique et succinique présents, puis par une recristallisation dudit acide adipique pour atteindre la pureté souhaitée. Lorque la séparation du catalyseur a été réalisée à l'aide d'une cristallisation, il est habituellement suffisant de pratiquer une recristallisation.

Ces cristallisation et/ou recristallisation peuvent être effectuées dans les conditions décrites précédemment. Elles consistent essentiellement à dissoudre l'acide adipique dans la quantité minimale d'eau, généralement à chaud, puis à cristalliser ou recristalliser ledit acide adipique par refroidissement de la solution dans les conditions habituelles de cristallisation ( par exemple diminution progressive programmée de la température, ensemencement par des cristaux d'acide adipique le cas échéant).

Les exemples qui suivent illustrent l'invention.

### EXEMPLES 1 ET 2

Dans un autoclave de 1,5 litre chemisé en titane et équipé d'une turbine six pales et de diverses ouvertures pour l'introduction des réactifs et des fluides ou pour l'évacuation des produits de la réaction et des fluides, que l'on a préalablement purgé à l'azote, on charge à température ambiante :
- acétate de cobalt tétrahydraté : 4,0 g (16 mmol)
- acide acétique : 357 g (5,95 mol)
- cyclohexane : 292,5 g (3,48 mol)
- cyclohexanone : 3,2 g (32,7 mmol).

Après fermeture de l'autoclave, la pression d'azote est portée à 20 bar, l'agitation est mise en route à 1000 tours/min et la température est amenée à 105°C en 20 min. L'azote est alors remplacé par 20 bar d'air appauvri (5 % d'oxygène). Le débit gazeux en entrée est réglé à 250 litres/heure.

Après une induction de 10 min environ, pendant laquelle il n'y a pas de consommation d'oxygène, la température s'élève de 2 à 3°C et l'oxygène commence à être consommé. Le titre en oxygène de l'air à l'entrée de l'autoclave est progressivement amené à 21 % en fonction de la consommation par l'oxydation.

Le titre en oxygène à la sortie du réacteur reste inférieure à 5 % durant la totalité de l'essai. La température dans l'autoclave oscille entre 104,9 et 105,1°C.

Lorsque 50 litres d'oxygène ont été consommés (taux de transformation du cyclohexane de 20 % environ), on commence l'injection en continu de la phase liquide : injection d'une solution d'acide acétique contenant 1,1 % en poids d'acétate de cobalt tétrahydraté à un débit de 3,7 ml/min et injection de cyclohexane à un débit de 4,1 ml/min. Le produit liquide est stocké en continu dans un décanteur de 7 litres à 70°C.

Après 400 minutes depuis le début de la réaction, l'air est progressivement remplacé par de l'azote, le contenu de l'autoclave est transféré dans le décanteur. Le contenu du décanteur est un mélange biphasique. La phase supérieure, essentiellement cyclohexanique qui contient peu de produits et de cobalt est séparée. La phase inférieure acétique (2340 g) contient l'essentiel des produits de l'oxydation et du cobalt. La phase acétique est soumise à une distillation en deux étapes dans les conditions suivantes :

### a) étape 1 de distillation :

- pression : 60 kPa
- température : 135°C.

Les résultats obtenus sont rassemblés dans le tableau ci-après.

Le distillat représente 1830 g et le pied de distillation environ 510 g.

### b) étape 2 de distillation.

Le pied de distillation issu de l'étape 1) est débarrassé des composés organiques volatils qu'il contient grâce à une injection de vapeur d'eau à 150 °C sous une pression de 10 kPa (743 g de vapeur en 7 h).

Les résultats obtenus sont rassemblés dans le tableau ci-après.

| **Composés** | **Masse initiale non traitéen** | **Pied de distillation étape 1** | **Pied de distillation étape 2** |
|---|---|---|---|
| cyclohexanone | 183,3 mmol | 75 mmol | 0 |
| acétate de cyclohexyle | 19,3 mmol | 36,3 mmol | 0 |
| cyclohexanol libre | 217,3 mmol | 56,5 mmol | 0 |
| acide glutarique* | 184,5 mmol | 184,5 mmol | 184,5 mmol |
| acide succinique* | 121,1 mmol | 121,1 mmol | 121,1 mmol |
| acide adipique* | 1656,5 mmol | 1656,5 mmol | 1656,5 mmol |
| acide hydroxycaproïque | 23,4 mmol | 23,mmol | 23 mmol |
| acide hydroxyadipique | 76 mmol | 76 mmol | 76 mmol |
| butyrolactone | 77,9 mmol | 64 mmol | 0 |
| valérolactone | 23,4 mmol | 10 mmol | 0 |
| acide acétique | 1830 g | non dosé | < 10 mmol |

| | | | |
|---|---|---|---|
| (*) : acide total (libre et estérifié) | | | |

On rajoute 1000 g d'eau au pied de distillation de l'étape 2. L'ensemble est chauffé à 70°C, puis est progressivement refroidi jusqu'à température ambiante selon le profil de température suivant : 12°C/h de 70°C à 60°C, 5°C/h de 60°C à 55°C, 11°C/h de 55°C à 44°C, 24°C/h de 44°C à 20°C.

Après filtration et lavages à l'eau, on obtient 200 g d'acide adipique brut, ayant une granulométrie moyenne de 300 µm et comportant (en poids par poids) :
- acide succinique : 0,2000 %
- acide glutarique : 0,0030 %
- cobalt : 0,0100 %.

Une recristallisation dans l'eau de 65 g de cet acide adipique brut conduit à un acide adipique (A) ayant une granulométrie moyenne de 300 µm et comportant (en poids par poids) :
- acide succinique : 0,0002 %
- acide glutarique : < 0,0001 %
- cobalt : < 0,0002 %.

Le catalyseur au cobalt se trouve dans les eaux de cristallisation.

65 g de l'acide adipique brut sont soumis au traitement par hydrogénation suivant.

Dans un autoclave de 500 ml, agité par secousses et chauffé par un four électrique, on introduit 65 g d'acide adipique brut obtenu précédemment à partir du pied de distillation de l'étape 2, 152 g d'eau et 2,8 g d'un catalyseur Pd/C à 10 % en poids de Pd. Après avoir purgé l'autoclave à température ambiante avec de l'azote, on le pressurise à 20 bar avec de l'hydrogène.

On chauffe à 135°C pendant 2h. On refroidit à 70°C, on dépressurise avec précaution et on filtre le catalyseur à cette température. On recristallise ensuite l'acide adipique comme précédemment pour l'acide (A). On obtient ainsi un acide adipique purifié (B) qui présente des caractéristiques très proches de l'acide adipique (A) en ce qui concerne les acides succinique et glutarique et le cobalt.

65 g de l'acide adipique brut sont soumis au traitement nitrique suivant.

On chauffe à 65°C 158 g d'acide nitrique à 52 % en poids. On rajoute en 10 min 32 g de l'acide adipique brut obtenu précédemment à partir du pied de distillation de l'étape 2. On ajoute alors 70 mg de nitrite de sodium. La température monte à 75°C. En maintenant cette température, on rajoute en 10 min 33 g de l'acide brut obtenu en pied de distillation de l'étape 2. On maintient en température pendant 1 h, puis on élimine les vapeurs nitreuses formées, par bullage d'azote pendant 30 min.

On recristallise ensuite l'acide adipique comme précédemment pour l'acide (A), puis on lave à l'eau jusqu'à neutralité des lavages. On obtient ainsi un acide adipique purifié (C) qui présente des caractéristiques très proches de l'acide adipique (A) en ce qui concerne les acides succinique et glutarique et le cobalt.

Les lots (A), (B) et (C) d'acide adipique sont soumis à un test de chauffage. Ce test consiste à chauffer 50 g de chaque lot à 215°C pendant 205 min, puis à placer chacun d'eux dans 415 ml d'une solution aqueuse d'ammoniaque à 5 %.

On mesure ensuite l'absorbance à 454 nm (zone du jaune ) des adipates d'ammonium obtenus.

On obtient les résultats suivants, exprimés en absorbance relative, l'acide adipique (A) de référence représentant la valeur 1 :
- acide adipique (A) : 1
- acide adipique (B) : 0,08
- acide adipique (C) : 0,12.

Les acides adipiques (B) et (C) purifiés selon la présente invention sont nettement moins colorés que l'acide adipique (A) non compris dans l'invention. L'absorbance à 454 nm est respectivement 12 et 8 fois moins importante pour (B) et (C) que pour (A), qui possède cependant déjà une excellente pureté par rapport aux diacides inférieurs ou au catalyseur (Co), mais qui s'avère contenir des traces plus importantes d'autres impuretés colorées.

## Revendications

1. Procédé de traitement du mélange réactionnel issu de l'oxydation directe du cyclohexane en acide adipique par l'oxygène moléculaire, dans un solvant organique et en présence d'un catalyseur, **caractérisé en ce que** ledit procédé comporte :
- une décantation en deux phases liquides : une phase supérieure essentiellement cyclohexanique et une phase inférieure comprenant essentiellement le solvant, les diacides formés, le catalyseur et une partie des autres produits de la réaction et du cyclohexane non transformé ;
- une distillation de ladite phase inférieure, permettant de séparer d'une part un distillat comprenant au moins une partie des composés les plus volatils tels que le solvant organique, l'eau ainsi que le cyclohexane non transformé, la cyclohexanone, le cyclohexanol, les esters de cyclohexyle et les lactones éventuellement présents, et d'autre part le pied de distillation comprenant les diacides formés, le catalyseur ;
- une séparation du catalyseur du pied de distillation obtenu précédemment, soit par cristallisation dans l'eau, par électrodialyse ou par passage sur résine échangeuse d'ions, après dissolution dudit pied de distillation dans l'eau, soit par lavage à l'eau ou par extraction liquide-liquide ;
- un traitement de purification réducteur et/ou oxydant de l'acide adipique en solution aqueuse ;
- une cristallisation précédant ou suivant le traitement de purification lorsqu'elle n'a pas été réalisée pour séparer le catalyseur ;
- une recristallisation dans l'eau de l'acide adipique.

2. Procédé selon la revendication 1, **caractérisé en ce que** la phase cyclohexanique obtenue dans l'étape de décantation est réintroduite dans une opération d'oxydation du cyclohexane.

3. Procédé selon l'une des revendications 1 ou 2, **caractérisé en ce que** le solvant organique mis en oeuvre dans l'oxydation du cyclohexane est choisi parmi les acides carboxyliques aliphatiques et est de préférence l'acide acétique.

4. Procédé selon l'une des revendications 1 à 3, **caractérisé en ce que** le catalyseur contient du cobalt, du manganèse, un mélange de cobalt avec un ou plusieurs autres métaux choisi parmi le manganèse, le chrome, le fer, le zirconium, l'hafnium, le cuivre.

5. Procédé selon l'une des revendications 1 à 4, **caractérisé en ce que** l'étape de distillation de la phase inférieure est conduite pour séparer de l'acide adipique, le cyclohexane non transformé et au moins 90 % du solvant encore présents dans cette phase inférieure.

6. Procédé selon l'une des revendications 1 à 5, **caractérisé en ce que** l'étape de distillation est effectuée à une température de 25°C à 250°C et sous une pression absolue comprise entre 10 Pa et la pression atmosphérique et de préférence à une température située entre 70°C et 150°C.

7. Procédé selon l'une des revendications 1 à 6, **caractérisé en ce que** l'étape de distillation est complétée par une extraction du pied de distillation à l'aide d'un solvant organique non miscible à l'eau.

8. Procédé selon la revendication 7, **caractérisé en ce que** l'extraction est effectuée avec un solvant organique choisi parmi les hydrocarbures aliphatiques, cycloaliphatiques ou aromatiques, les esters d'acides carboxyliques aliphatiques, cycloaliphatiques ou aromatiques et les cétones, et de préférence avec le cyclohexane.

9. Procédé selon l'une des revendications 1 à 8, **caractérisé en ce que** le pied de distillation obtenu en fin de distillation, et ayant subi le cas échéant l'opération d'extraction, est traité pour séparer le catalyseur qu'il contient, par une opération de cristallisation ou par une électrodialyse ou en passant ladite solution sur une résine échangeuse d'ions ou par un ou plusieurs lavages à l'eau.

10. Procédé selon l'une des revendications 1 à 9, **caractérisé en ce que** la purification est réalisée par hydrogénation et/ou par traitement à l'acide nitrique et/ou par oxydation à l'aide d'oxygène moléculaire, d'ozone ou d'hydroperoxyde.

11. Procédé selon la revendication 10, **caractérisé en ce que** la purification par hydrogénation est effectuée à l'aide d'hydrogène et en présence d'un catalyseur.

12. Procédé selon l'une des revendications 10 ou 11, **caractérisé en ce que** le catalyseur contient au moins un métal du groupe 8 de la Classification périodique des éléments tel que le palladium, le platine, le ruthénium, l'osmium, le rhodium, l'iridium, le nickel et le cobalt, de préférence déposé sur un support solide.

13. Procédé selon la revendication 10, **caractérisé en ce que** la purification par traitement à l'acide nitrique est réalisée avec une solution aqueuse contenant de 20 % à 80 % d'acide nitrique pur en poids par poids de solution.

14. Procédé selon la revendication 13 **caractérisé en ce que** le traitement à l'acide nitrique est effectué en chauffant le mélange à une température de 25°C à 120°.

15. Procédé selon l'une des revendications 13 ou 14, **caractérisé en ce que** le traitement à l'acide nitrique est réalisé en l'absence de catalyseur ou en présence d'un catalyseur, comprenant un ou plusieurs composés de cobalt, de cuivre et/ou de vanadium.

16. Procédé selon la revendication 10, **caractérisé en ce que** la purification par oxydation est réalisée avec de l'air, de l'air enrichi en oxygène ou de l'air appauvri en oxygène et en présence d'un catalyseur.

17. Procédé selon la revendication 16, **caractérisé en ce que** le catalyseur est un métal du groupe 8 de la Classification périodique des éléments choisi parmi le palladium, le platine, le ruthénium, l'osmium, le rhodium, l'iridium.

18. Procédé selon la revendication 10, **caractérisé en ce que** la purification par oxydation à l'aide d'un hydroperoxyde est réalisée avec le peroxyde d'hydrogène.

19. Procédé selon l'une des revendications 1 à 18, **caractérisé en ce que** le traitement de purification réducteur et/ou oxydant est suivi par une opération de cristallisation et/ou de recristallisation de l'acide adipique dans l'eau.

20. Procédé selon l'une des revendications 1 à 19, **caractérisé en ce que** le traitement de purification réducteur et/ou oxydant est précédé ou est suivi par un traitement d'adsorption d'impuretés par un solide finement divisé.

## Patentansprüche

1. Verfahren zur Behandlung einer Reaktionsmischung, die aus der direkten Oxidation von Cyclohexan zu Adipinsäure durch molekularen Sauerstoff in einem organischen Lösungsmittel und in Anwesenheit eines Katalysators stammt, **dadurch gekennzeichnet, daß** das genannte Verfahren umfaßt:
- eine Dekantierung in zwei flüssige Phasen : eine obere, im wesentlichen cyclohexanische Phase und eine untere Phase, die im wesentlichen das Lösungsmittel, die gebildeten Disäuren, den Katalysator und einen Teil der anderen Reaktionsprodukte sowie nicht umgesetztes Cyclohexan umfaßt;
- eine Destillation der genannten unteren Phase, die die Abtrennung ermöglicht von einerseits einem Destillat, das mindestens einen Teil der flüchtigsten Verbindungen wie das Lösungsmittel, Wasser sowie das nicht umgesetzte Cyclohexan, Cyclohexanon, Cyclohexanol, die Cyclohexyl-ester und die gegebenenfalls anwesenden Lactone umfaßt, und andererseits einem Destillationssumpf, der die gebildeten Disäuren und den Katalysator umfaßt;
- eine Abtrennung des Katalysators von dem vorstehend erhaltenen Destillationssumpf, entweder durch Kristallisation in Wasser, durch Elektrodialyse oder durch Passage über Ionenaustauscherharze, nach der Auflösung des genannten Destillationssumpfes in Wasser, oder durch Waschen mit Wasser oder durch Extraktion flüssig-flüssig;
- eine Behandlung zur reduzierenden und/oder oxidierenden Reinigung der Adipinsäure in wäßriger Lösung;
- eine Kristallisation vor oder nach der Behandlung zur Reinigung, wenn sie nicht zur Abtrennung des Katalysators durchgeführt wurde;
- eine Rekristallisation der Adipinsäure in Wasser.

2. Verfahren nach Anspruch 1, **dadurch gekennzeichnet, daß** die in der Stufe der Dekantierung erhaltene cyclohexanische Phase in eine Operation zur Oxidation des Cyclohexans zurückgeführt wird.

3. Verfahren nach feinem der Ansprüche 1 oder 2, **dadurch gekennzeichnet, daß** das bei der Oxidation des Cyclohexans eingesetzte Lösungsmittel unter den aliphatischen Carbonsäuren ausgewählt wird und vorzugsweise Essigsäure ist.

4. Verfahren nach einem der Ansprüche 1 bis 3, **dadurch gekennzeichnet, daß** der Katalysator Cobalt, Mangan, eine Mischung von Cobalt mit einem oder mehreren anderen Metallen enthält, ausgewählt unter Mangan, Chrom, Eisen, Zirkonium, Hafnium, Kupfer.

5. Verfahren nach einem der Ansprüche 1 bis 4, **dadurch gekennzeichnet, daß** die Stufe der Destillation der unteren Phase durchgeführt wird, um die Adipinsäure, das nicht umgesetzte Cyclohexan und mindestens 90 % des noch in der unteren Phase anwesenden Lösungsmittels abzutrennen.

6. Verfahren nach einem der Ansprüche 1 bis 5, **dadurch gekennzeichnet, daß** die Stufe der Destillation bei einer Temperatur von 25 °C bis 250 °C und unter einem absolutem Druck zwischen 10 Pa und dem atmosphärischen Druck durchgeführt wird, und vorzugsweise bei einer Temperatur zwischen 70 °C und 150 °C.

7. Verfahren nach einem der Ansprüche 1 bis 6, **dadurch gekennzeichnet, daß** die Stufe der Destillation durch eine Extraktion des Destillationssumpfes mit Hilfe eines mit Wasser nicht mischbaren organischen Lösungsmittels vervollständigt wird.

8. Verfahren nach Anspruch 7, **dadurch gekennzeichnet, daß** die Extraktion mit einem organischen Lösungsmittel, ausgewählt unter den aliphatischen, cycloaliphatischen oder aromatischen Kohlenwasserstoffen, den Estern von aliphatischen, cycloaliphatischen oder aromatischen Carbonsäuren und den Ketonen, und vorzugsweise mit Cyclohexan durchgeführt wird.

9. Verfahren nach einem der Ansprüche 1 bis 8, **dadurch gekennzeichnet, daß** der am Ende der Destillation erhaltene Destillationssumpf, und der gegebenenfalls einer Operation zur Extraktion unterzogen wurde, behandelt wird, um den Katalysator zu entfernen, den er enthält, und zwar durch eine Operation zur Kristallisation oder durch eine Elektrodialyse oder durch Passage der genannten Lösung über ein Ionenaustauscherharz oder durch eine oder mehrere Waschungen mit Wasser.

10. Verfahren nach einem der Ansprüche 1 bis 9, **dadurch gekennzeichnet, daß** die Reinigung durch Hydrierung und/oder durch Behandlung mit Salpetersäure und/oder durch Oxidation mit Hilfe von molekularem Sauerstoff, Ozon oder Wasserstoffperoxid realisiert wird.

11. Verfahren nach Anspruch 10, **dadurch gekennzeichnet, daß** die Reinigung durch Hydrierung mit Hilfe von Wasserstoff und in Anwesenheit eines Katalysators durchgeführt wird.

12. Verfahren nach einem der Ansprüche 10 oder 11, **dadurch gekennzeichnet, daß** der Katalysator mindestens ein Metall der Gruppe VIII des Periodensystems der Elemente enthält, wie Palladium, Platin, Ruthenium, Osmium, Rhodium, Iridium, Nickel und Cobalt, vorzugsweise aufgebracht auf einem festen Träger.

13. Verfahren nach Anspruch 10, **dadurch gekennzeichnet, daß** die Reinigung durch Behandlung mit Salpetersäure mit einer wäßrigen Lösung realisiert wird, die 20 % bis 80 % reine Salpetersäure in Gewicht pro Gewicht der Lösung enthält.

14. Verfahren nach Anspruch 13, **dadurch gekennzeichnet, daß** die Behandlung mit Salpetersäure unter Erhitzen der Mischung auf eine Temperatur von 25 °C bis 120 °C durchgeführt wird.

15. Verfahren nach einem der Ansprüche 13 oder 14, **dadurch gekennzeichnet, daß** die Behandlung mit Salpetersäure bei Abwesenheit oder in Anwesenheit eines Katalysators realisiert wird, der eine oder mehrere Verbindungen von Cobalt, Kupfer und/oder Vanadium umfaßt.

16. Verfahren nach Anspruch 10, **dadurch gekennzeichnet, daß** die Reinigung durch Oxidation mit Luft, mit an Sauerstoff angereicherter Luft oder an Sauerstoff abgereicherter Luft und in Anwesenheit eines Katalysators realisiert wird.

17. Verfahren nach Anspruch 16, **dadurch gekennzeichnet, daß** der Katalysator ein Metall der Gruppe VIII des Periodensystems der Elemente ist, ausgewählt unter Palladium, Platin, Ruthenium, Osmium, Rhodium, Iridium.

18. Verfahren nach Anspruch 10, **dadurch gekennzeichnet, daß** die Reinigung durch Oxidation mit Hilfe eines Hydroperoxides mit Wasserstoffperoxid realisiert wird.

19. Verfahren nach einem der Ansprüche 1 bis 18, **dadurch gekennzeichnet, daß** die Behandlung zur reduzierenden und/oder oxidierenden Reinigung von einer Operation der Kristallisation und/ oder der Rekristallisation der Adipinsäure in Wasser gefolgt wird.

20. Verfahren nach einem der Ansprüche 1 bis 19, **dadurch gekennzeichnet, daß** der Behandlung zur reduzierenden und/oder oxidierenden Reinigung eine Behandlung zur Adsorption von Verunreinigungen durch einen feinverteilten Feststoff vorangeht oder ihr folgt.

## Claims

1. Process for the treatment of the reaction mixture resulting from the direct oxidation of cyclohexane to adipic acid by molecular oxygen in an organic solvent in the presence of a catalyst, **characterized in that** the said process comprises:
- a separation into two liquid phases by settling: an upper phase, which is essentially cyclohexane, and a lower phase, essentially comprising the solvent, the diacids formed, the catalyst and a portion of the other reaction products and of the unconverted cyclohexane;
- a distillation of the said lower phase, making it possible to separate, on the one hand, a distillate comprising at least a portion of the most volatile compounds, such as the organic solvent and water, as well as unconverted cyclohexane, cyclohexanone, cyclohexanol, cyclohexyl esters and lactones possibly present, and, on the other hand, the distillation bottoms comprising the diacids formed and the catalyst;
- a separation of the catalyst from the distillation bottoms obtained above, either by crystallization from water, by electrodialysis or by passing over an ionexchange resin, after dissolution of the said distillation bottoms in water, or alternatively by washing with water or by liquid-liquid extraction;
- a reducing and/or oxidizing purification treatment of the adipic acid in aqueous solution;
- a crystallization, preceding or following the purification treatment, when the crystallization has not been carried out in order to separate the catalyst;
- a recrystallization of the adipic acid from water.

2. Process according to claim 1, **characterized in that** the cyclohexane phase obtained in the stage of separation by settling is reintroduced into a cyclohexane oxidation operation.

3. Process according to either of claims 1 and 2, **characterized in that** the organic solvent employed in the oxidation of the cyclohexane is chosen from aliphatic carboxylic acids and is preferably acetic acid.

4. Process according to one of claims 1 to 3, **characterized in that** the catalyst comprises cobalt, manganese or a mixture of cobalt with one or more other metals chosen from manganese, chromium, iron, zirconium, hafnium or copper.

5. Process according to one of claims 1 to 4, **characterized in that** the stage of distillation of the lower phase is carried out so as to separate from the adipic acid, the unconverted cyclohexane and at least 90% of the solvent which are still present in this lower phase.

6. Process according to one of claims 1 to 5, **characterized in that** the distillation stage is carried out at a temperature of 25°C to 250°C and under an absolute pressure of between 10 Pa and atmospheric pressure and preferably at a temperature situated between 70°C and 150°C.

7. Process according to one of claims 1 to 6, **characterized in that** the distillation stage is completed by an extraction of the distillation bottoms using a water-immiscible organic solvent.

8. Process according to claim 7, **characterized in that** the extraction is carried out with an organic solvent chosen from aliphatic, cycloaliphatic or aromatic hydrocarbons, aliphatic, cycloaliphatic or aromatic carboxylic acid esters, and. ketones, and preferably with cyclohexane.

9. Process according to one of claims 1 to 8, **characterized in that** the distillation bottoms obtained at the end of the distillation, which have been subjected, if appropriate, to the extraction operation, are treated, in order to separate the catalyst which they comprise, by a crystallization operation or by an electrodialysis or by passing the said solution over an ionexchange resin or by one or more washing operations with water.

10. Process according to one of claims 1 to 9, **characterized in that** the purification is carried out by hydrogenation and/or by treatment with nitric acid and/or by oxidation using molecular oxygen, ozone or hydroperoxide.

11. Process according to claim 10, **characterized in that** the purification by hydrogenation is carried out using hydrogen in the presence of a catalyst.

12. Process according to either of claims 10 and 11, **characterized in that** the catalyst comprises at least one metal from group 8 of the Periodic Classification of the Elements, such as palladium, platinum, ruthenium, osmium, rhodium, iridium, nickel and cobalt, preferably deposited on a solid support.

13. Process according to claim 10, **characterized in that** the purification by treatment with nitric acid is carried out with an aqueous solution comprising from 20% to 80% of pure nitric acid by weight per weight of solution.

14. Process according to claim 13, **characterized in that** the treatment with nitric acid is carried out by heating the mixture at a temperature of 25°C to 120°C.

15. Process according to either of claims 13 and 14, **characterized in that** the treatment with nitric acid is carried out in the absence of catalyst or in the presence of a catalyst comprising one or more cobalt, copper and/or vanadium compounds.

16. Process according to claim 10, **characterized in that** the purification by oxidation is carried out with air, air enriched in oxygen or air depleted in oxygen, in the presence of a catalyst.

17. Process according to claim 16, **characterized in that** the catalyst is a metal from group 8 of the Periodic Classification of the Elements chosen from palladium, platinum, ruthenium, osmium, rhodium or iridium.

18. Process according to claim 10, **characterized in that** the purification by oxidation using a hydroperoxide is carried out with hydrogen peroxide.

19. Process according to one of claims 1 to 18, **characterized in that** the reducing and/or oxidizing purification treatment is followed by an operation of crystallization and/or of recrystallization of the adipic acid from water.

20. Process according to one of claims 1 to 19, **characterized in that** the reducing and/or oxidizing purification treatment is preceded or is followed by a treatment for adsorption of impurities by a finely divided solid.
